**Europäisches Patentamt**

**European Patent Office**

**Office européen des brevets**

(19)

(11) Numéro de publication : **0 115 893**
**B1**

(12)

# FASCICULE DE BREVET EUROPÉEN

(45) Date de publication du fascicule du brevet :
12.11.86

(51) Int. Cl.⁴ : **A 61 L   2/02// A01N59/00**

(21) Numéro de dépôt : **84200133.1**

(22) Date de dépôt : **01.02.84**

(54) **Appareil pour stériliser des objets à l'aide d'une solution aqueuse d'hypochlorite.**

(30) Priorité : **03.02.83 CH 609/83**

(43) Date de publication de la demande :
**15.08.84 Bulletin 84/33**

(45) Mention de la délivrance du brevet :
**12.11.86 Bulletin 86/46**

(84) Etats contractants désignés :
**AT BE CH DE FR GB IT LI LU NL SE**

(56) Documents cités :
**US-A- 3 819 329**

(73) Titulaire : **BATTELLE MEMORIAL INSTITUTE**
**7 route de Drize**
**CH-1227 Carouge/Genève (CH)**

(72) Inventeur : **Porta, Augusto**
**52, route de Drize**
**CH-1227 Carouge (CH)**
Inventeur : **Schaub, Maurice**
**Lieu dit Le Fougueux**
**F-74550 Perrignier (FR)**
Inventeur : **Ayerbe, André**
**Les Chavannes Ramboex**
**F-74800 Reignier (FR)**
Inventeur : **Bunter, Guy**
**28, avenue Vibert**
**CH-1227 Carouge (CH)**

(74) Mandataire : **Dousse, Blasco et al**
**7, route de Drize**
**CH-1227 Carouge/Genève (CH)**

**0 115 893**

## Description

La présente invention se rapporte à un appareil pour stériliser des objets à l'aide d'une solution aqueuse d'hypochlorite.

Il existe sur le marché un récipient, ainsi qu'un produit, constitué d'une solution aqueuse d'hypochlorite pour la stérilisation des biberons et des tétines. Ce récipient est conçu pour recevoir les biberons et les tétines immergés dans la solution d'hypochlorite durant un minimum de 90 minutes, après quoi les biberons sont rincés à l'eau bouillie, afin d'enlever le goût donné par cette solution. Pour obtenir une stérilisation convenable en 90 minutes, la concentration de la solution en hypochlorite doit être de 125 ppm. Cette durée peut difficilement être réduite en augmentant la concentration sans s'exposer à ce que le goût de l'hypochlorite subsiste après le rinçage.

On a déjà proposé, dans la demande de brevet WO 81/01863, une machine à laver le linge ou la vaisselle équipée d'une cellule électro-chimique destinée à produire une solution d'hypochlorite à concentration désirée en vue d'exercer une action blanchissante ou décolorante. A cet effet, la solution d'hypochlorite est introduite dans le bain de lavage lorsqu'elle a atteint une certaine concentration et, à l'instant choisi, au cours du cycle de lavage.

Des essais ont été réalisés avec des solutions aqueuses d'hypochlorite fraîchement produites dans une cellule électro-chimique et introduites à concentration désirée dans un bain dans lequel des biberons ont été immergés. Ces essais ont montré que l'effet de l'hypochlorite en solution aqueuse fraîchement fabriquée dans une cellule électro-chimique est similaire à celui de l'hypochlorite vendu pour le récipient de stérilisation des biberons.

Par contre, des résultats surprenants ont été constatés lorsque les biberons sont traités par la solution d'hypochlorite en cours de production en prélevant de cette solution pour la faire passer sur les surfaces à stériliser et en la ramenant dans la cellule électro-chimique de production d'hypochlorite. La concentration de cette solution en hypochlorite croît au fur et à mesure du déroulement du processus.

Des tests comparatifs, dont on donnera les détails par la suite, ont démontré que la destruction de germes est obtenue par ce mode de traitement en un temps sensiblement plus court et avec une concentration plus faible de NaOCl que dans le cas de l'immersion des biberons dans une solution de NaOCl obtenue par adjonction à l'eau d'une solution concentrée de NaOCl produite préalablement au processus de stérilisation. On pourrait peut-être expliquer ce résultat surprenant par le fait que l'utilisation de la solution de NaOCl pendant sa production bénéficie du chlore produit au cours du processus électro-chimique et qui ne se combine pas totalement et instantanément pour produire le NaOCl. Or on connaît l'action antiseptique du chlore, qui, une fois combiné sous forme d'hypochlorite, aurait une action antiseptique atténuée. Cette hypothèse expliquerait la raison pour laquelle on constate une action beaucoup plus rapide avec une solution à concentration extrêmement faible en NaOCl.

On a déjà proposé dans le US-A-3.819.329 un système dans lequel une solution bactéricide à relativement bas pH contenant du chlore naissant, pratiquement entièrement sous la forme d'acide hypochloreux est engendré dans une cellule électro-chimique et distribué ensuite sur les surfaces à stériliser. La solution est donc produite à la concentration désirée dans la cellule puis elle est distribuée. Par conséquent, la production et l'utilisation de la solution ne sont pas simultanées. Or les tests comparatifs montrent l'importance de l'utilisation pendant la production du NaOCl pour augmenter l'efficacité de la stérilisation, ce que ne permet pas de faire le système proposé par ce document.

Dans le GB-A-2.094.992 des objets à stériliser, notamment des lentilles de contact, sont trempés dans une solution physiologique qui est électrolysée afin d'obtenir une solution de NaOCl. Compte tenu de la dimension extrêmement réduite des lentilles de contact, aucun moyen de distribution n'est prévu. Etant donné le très faible volume de solution nécessaire, celle-ci est produite uniquement en disposant deux électrodes dans la cellule. Si une telle solution est acceptable compte tenu du très faible volume de solution à produire, elle ne l'est en revanche pas lorsque ce volume est sensiblement plus important étant donné qu'en l'absence d'agitation de la solution en cours d'électrolyse, le rendement faradique diminue rapidement en raison du phénomène de polarisation des électrodes.

Le but de la présente invention est de mettre en œuvre un processus de stérilisation d'objets par une solution d'hypochlorite au fur et à mesure de sa production par une cellule électro-chimique.

A cet effet, la présente invention a pour objet un appareil pour stériliser des objets à l'aide d'une solution aqueuse d'hypochlorite selon la revendication 1.

La solution proposée présente l'avantage d'utiliser l'hypochlorite au fur et à mesure de sa production. Etant donné que le NaOCl est circulé en circuit fermé pendant sa production en arrosant les surfaces à stériliser, la quantité de solution à produire est considérablement réduite. En outre, cette circulation en circuit fermé de la solution de NaOCl produite crée simultanément un brassage du liquide dans la cellule et assure ainsi un rendement faradique élevé tout au long du processus de production.

Outre l'important gain de temps, l'appareil objet de l'invention permet de stériliser avec des concentrations encore plus faibles d'hypochlorite diminuant le goût résiduel sur les objets stérilisés et réduisant le rinçage nécessaire lorsqu'il s'agit de stériliser des objets destinés à contenir des aliments ou des boissons tels que des biberons ou des bouteilles.

Compte tenu de la rapidité du processus mis en œuvre à l'aide de l'appareil objet de l'invention, cet

2

appareil n'est pas limité à la stérilisation de biberons, mais il est tout à fait possible d'envisager son adaptation pour la stérilisation de bouteilles ou autres récipients destinés à l'industrie alimentaire, pharmaceutique ou cosmétique par exemple.

A l'heure actuelle, la stérilisation est obtenue thermiquement par le passage dans des fours ou par l'injection de vapeur d'eau. Ces modes de stérilisation sont coûteux et l'appareil objet de l'invention trouve également dans ce cas une application qui permet de réaliser une importante économie d'énergie.

Le dessin annexé illustre, schématiquement et à titre d'exemple, une forme d'exécution de l'appareil à stériliser objet de la présente invention.

La figure 1 est une vue en perspective de cet appareil.

La figure 2 est une vue en perspective d'un détail représenté par la fig. 1.

Bien que l'appareil illustré par cet exemple, que nous allons décrire, se rapporte à un appareil à stériliser les biberons, il est évidemment à la portée de l'homme de métier d'adapter cet appareil pour stériliser d'autres objets, de même qu'il est possible d'adapter cet appareil pour le traitement en continu de bouteilles dans une installation de lavage et de stérilisation de bouteilles ou d'autres récipients destinés à recevoir des aliments, des boissons, des produits pharmaceutiques ou des produits cosmétiques par exemple.

Cet appareil comporte une enceinte 1 posée sur un socle 2. Cette enceinte renferme deux électrodes 3 et 4 disposées horizontalement l'une au-dessus de l'autre dans le fond de l'enceinte 1. Ces électrodes sont destinées à produire une solution aqueuse de NaOCl par électrolyse d'une solution aqueuse de NaCl, et sont reliées à cet effet à une source de courant continu obtenue après transformation et redressement du courant du réseau par un transformateur redresseur 16. Des supports de positionnement 5, recevant des biberons 9 à stériliser en position retournée sont constitués chacun par quatre ailettes disposées radialement par rapport à un axe central et à 90° les unes des autres et sont placées sur une tubulure de distribution 6 de la solution d'hypochlorite produite par les électrodes 3 et 4. Cette tubulure est reliée à la sortie d'une pompe de circulation 7 de la solution de NaOCl, dont l'admission est reliée au fond de l'enceinte 1. Cette tubulure comporte quatre conduits de distribution verticaux 8, dont seul l'un est représenté, s'étendant le long de l'axe vertical situé au centre des ailettes de chaque support de positionnement 5. Pour des raisons de clarté, seuls deux supports de positionnement ont été représentés. Les biberons 9 destinés à être placés sur les deux supports de positionnement non dessinés montrent l'emplacement de ces deux supports. Les conduits de distribution verticaux 8 sont prolongés chacun par une canne 10 dont l'extrémité se termine par une pomme de douche 11 destinée à distribuer la solution sous pression sous forme d'une pluralité de jets dirigés vers le fond de chaque biberon 9.

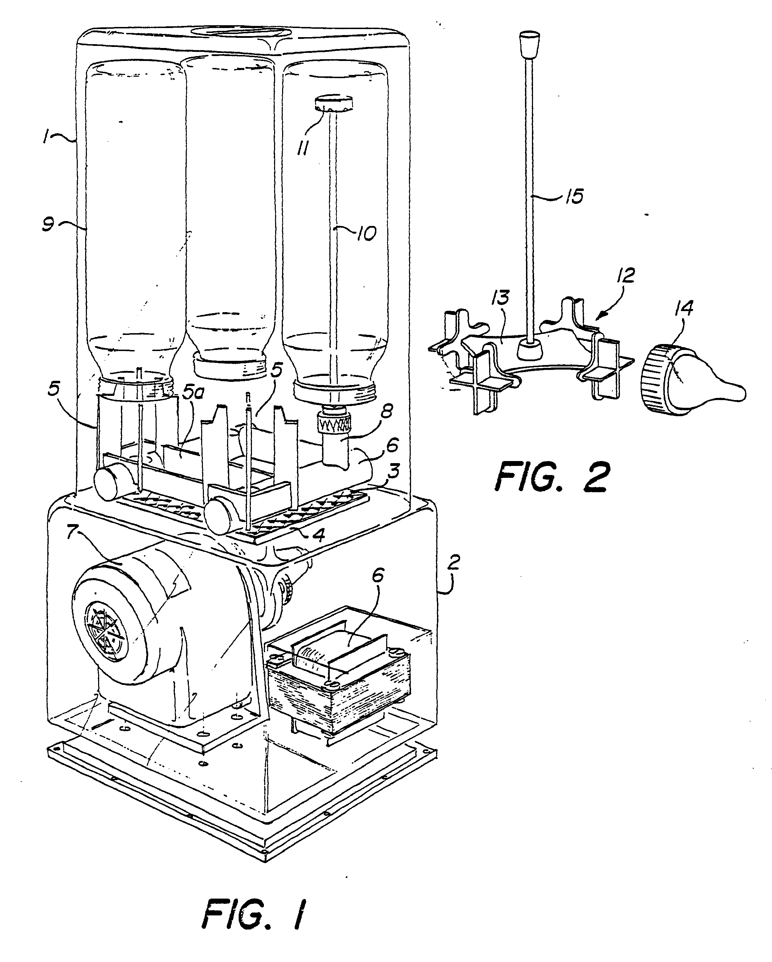

Un support de tétines 12 (fig. 2) en forme d'étoile à quatre branches 13, dont les extrémités respectives sont destinées à recevoir les tétines 14, est muni d'une tige verticale 15 permettant de le manipuler pour le mettre et l'enlever de l'enceinte 1. Les branches 13 de l'étoile sont destinées à prendre place sur la partie de liaison 5a reliant les supports de positionnement 5 des biberons 9 et donc entre ces biberons, à proximité du fond de l'enceinte 1. Ces tétines 14 sont disposées avec leurs axes de révolution respectifs horizontaux pour ne pas emprisonner de bulles d'air.

Pour stériliser les biberons à l'aide de cet appareil, on dispose le support de tétine 12 et les biberons 9 dans l'enceinte 1, comme illustré. On introduit dans l'enceinte 1, 800 cc d'eau et 6 g de NaCl, cette solution arrivant au niveau du pas de vis des biberons et recouvrant les tétines. On relie les électrodes 3 et 4 au transformateur redresseur 16 et, simultanément, on met en marche la pompe de circulation 7 distribuant la solution à l'intérieur des biberons 9 au fur et à mesure que la solution de NaCl est transformée par électrolyse en NaOCl. Si nécessaire, on peut aussi stériliser les surfaces extérieures des biberons en prévoyant une aspersion de leurs faces externes.

Une série de tests comparatifs ont été réalisés pour tester l'efficacité de la stérilisation avec l'appareil décrit. Le test de base servant de comparaison a été réalisé à l'aide d'une solution de NaOCl à 125 ppm qui correspond à la concentration utilisée dans l'appareil de stérilisation de biberons que l'on trouve sur le marché. L'eau a été contaminée en colibacilles du type Escherichia coli à une densité de $10^8$/ml. Le traitement par immersion a duré 90 minutes selon indication d'utilisation à température ambiante de l'ordre de 20 °C, le pH de cette solution étant de 8,7. La stérilisation obtenue est complète. En réduisant quelque peu la concentration de la solution à 100 ppm pour une durée d'immersion de 90 minutes, on relève une contamination résiduelle, ce qui démontre que les conditions énoncées dans le test précédent sont des conditions limites pour l'obtention d'une stérilisation totale.

Des tests ont été effectués pour déterminer les limites d'utilisation de l'appareil décrit en partant de la même densité de colibacilles du type Escherichia coli à $10^8$/ml. Le tableau ci-dessous donne les résultats obtenus en traitant par aspersion simultanément quatre biberons et leurs tétines.

(Voir tableau p. 4)

On constate que la stérilisation totale est obtenue de façon répétitive pour un courant de l'ordre de 0,5 à 1A appliqué pendant environ 10 minutes. Simultanément à l'aspersion de la face interne des biberons, la concentration de la solution en NaOCl augmente pour atteindre à la fin du traitement une valeur de l'ordre de 30 ppm, au lieu de 125 ppm, alors que la durée du traitement est neuf fois plus courte.

Par contre, les essais réalisés par immersion dans une solution d'hypochlorite, produite séparément

| Intensité du courant en A | Durée de traitement en minutes | Résultat de la stérilisation |
|---|---|---|
| 0,5 | 15 | 0 |
| 0,5 | 10 | 0 |
| 0,5 | 10 | légèrement contaminé |
| 0,5 | 10 | 0 |
| 0,5 | 1 | contaminé |
| 0,5 | 2 | 0 |
| 0,25 | 5 | contaminé |
| 0,25 | 6 | 0 |

par voie électro-chimique, même utilisé tout de suite après son obtention, ont donné des résultats en tout point similaire aux tests de référence susmentionnés réalisés avec l'appareil et la solution de NaOCl vendus dans le commerce pour stériliser les biberons.

Ceci montre que ce n'est pas le mode de production du NaOCl qui joue un rôle, mais le fait de pouvoir utiliser cette solution de NaOCl pendant sa production électro-chimique. La conception de l'appareil objet de l'invention est précisément étudiée en vue de permettre la production de NaOCl et sa mise en contact simultanée avec les surfaces à stériliser des biberons et des tétines.

Un autre avantage de l'invention réside dans la circulation de la solution de stérilisation contre les surfaces à stériliser, ce qui permet de réduire considérablement le volume de la solution et, par conséquent, la durée nécessaire pour atteindre la concentration désirée. En outre, la solution étant produite au fur et à mesure, il est important de la faire circuler, ce qui ne se produit pas dans le cas d'une simple immersion.

## Revendications

1. Appareil pour stériliser des objets à l'aide d'une solution aqueuse d'hypochlorite, comprenant une cellule de production électro-chimique d'hypochlorite à partir d'une solution de NaCl caractérisé par le fait qu'il comporte des moyens pour faire circuler en continu cette solution, en cours de production de l'hypochlorite, entre ladite cellule et les surfaces à stériliser de ces objets et pour la ramener vers cette cellule.

2. Appareil selon la revendication 1, caractérisé par le fait que ladite cellule comporte deux électrodes destinées à être reliées à une source de courant continu, disposées au fond d'une enceinte comprenant des supports de positionnement des objets à stériliser disposés au-dessus de ces électrodes, une tubulure de distribution de la solution contre les surfaces à stériliser de ces objets, non immergées dans ladite solution et une pompe de circulation dont l'admission est reliée au fond de ladite enceinte et la sortie est connectée à ladite tubulure de distribution de la solution.

## Claims

1. Apparatus for sterilizing objects by means of an aqueous hypochlorite solution, comprising an electrochemical cell for the production of hypochlorite from a NaCl solution, characterized in that it comports means for continuously circulating this solution, during the production of hypochlorite, between said cell and the surfaces of these objects to be sterilized and to bring it back to this cell.

2. Apparatus according to claim 1, characterized in that said cell comports two electrodes to be connected to a direct current power source housed at the bottom of an enclosure comprising brackets for positioning objects to be sterilized above these electrodes, a duct for distributing the solution onto the surfaces of these objects to be sterilized, these not being immersed in the solution, and a circulation pump whose input is connected to the bottom of said enclosure and whose output is connected to said solution distribution duct.

## Patentansprüche

1. Vorrichtung zum Sterilisieren von Gegenständen mit Hilfe einer wässerigen Hypochloritlösung, mit einer Zelle zur elektrochemischen Erzeugung von Hypochlorit aus einer NaCl-lösung, dadurch

gekennzeichnet, daß sie Mittel zum kontinuierlichen Zirkulierenlassen dieser Lösung während der Herstellung des Hypochlorites zwischen der Zelle und den zu sterilisierenden Flächen dieser Gegenstände und zur Rückführung zu dieser Zelle aufweist.

2. Vorrichtung nach Anspruch 1, dadurch gekennzeichnet, daß die Zelle zwei Elektroden aufweist, die mit einer Gleichstromquelle verbindbar und am Boden eines Behälters angeordnet sind, der Träger zur Lageanordnung der zu sterilisierenden Gegenstände oberhalb dieser Elektroden, ein Rohrleitungssystem zur Verteilung der Lösung auf den zu sterilisierenden Flächen dieser Gegenstände, die nicht in die Lösung eintauchen, und eine Zirkulationspumpe umfaßt, deren Einlaß mit dem Boden des Behälters und dessen Ausgang mit dem Rohrleitungssystem zur Verteilung der Lösung verbunden sind.

FIG. 2

FIG. 1

1